# EUROPEAN PATENT APPLICATION

(11) **EP 4 316 245 A1**
(43) Date of publication of application: **07.02.2024**
(21) Application number: 22779710.7
(22) Date of filing: 25.02.2022
(51) Int. Cl.: A01N 61/00, A01H 3/00, A01N 61/02, A01P 21/00

(54) **AGENT FOR INCREASING EXPRESSION OF GENE AND PHOTOSYNTHESIS ACTIVATING AGENT**

(30) Priority: 29.03.2021 JP 2021055089
(71) Applicant: Denka Company Limited, Tokyo 103-8338 (JP); Tohoku University, Sendai-shi, Miyagi 980-8577 (JP)
(72) Inventor: OKAWA Takashi, Tokyo 103-8338 (JP); HONDA Kazuma, Tokyo 103-8338 (JP); IINO Toju, Tokyo 103-8338 (JP); KATO Kazuhisa, Sendai-shi, Miyagi 980-8577 (JP)
(74) Representative: Gulde & Partner
(86) International application number: PCT/JP2022/007914
(87) International publication number: WO 2022/209485

(57) **Abstract**

Disclosed is an agent for increasing the expression of a stress response gene in a plant, the agent containing a humic acid.

## Description

### Technical Field

The present invention relates to an expression enhancer for a stress responsive gene and a method for imparting stress resistance to a plant. The present invention also relates to an expression enhancer for a gene involved in nitrogen metabolism. The present invention further relates to a photosynthesis activator and a method for activating photosynthesis of a plant.

### Background Art

Plants are sensitive to various stresses such as drought, salts, diseases, pests, and temperature. Means for improving stress response of plants has been investigated, and for example, Patent Literature 1 discloses a method of bringing a composition into contact with a plant, wherein the composition comprises an agriculturally acceptable complex mixture of dissolved organic substances characterized by being partially decomposed natural organic substances. Furthermore, in Patent Literature 1, such a composition is characterized by being a unique composition chemically and biologically different from fulvic acid and humic acid.

### Citation List

### Patent Literature

Patent Literature 1: JP 2016-154533 A

### Summary of Invention

### Technical Problem

An object of the present invention is to provide means for improving stress response of a plant, and more specifically, it is an object of the present invention to provide an expression enhancer for a stress responsive gene.

### Solution to Problem

The inventors of the present invention conducted a thorough investigation in order to solve the above-described objects, and they found that humic substances enhance the expression of a stress responsive gene of a plant. Furthermore, the present inventors further conducted investigations and found that humic substances also enhance the expression of a gene involved in nitrogen metabolism of a plant and that humic substances increase the photosynthetic rate, thus completing the present invention.

That is, the present invention relates to the following [1] to [20].
[1] An expression enhancer for a stress responsive gene of a plant, comprising humic substances.
[2] The expression enhancer according to the above-described [1], wherein humic substances are derived from lignite.
[3] The expression enhancer according to the above-described [1] or [2], wherein the melanic index is 2.0 or higher.
[4] The expression enhancer according to any one of the above-described [1] to [3], wherein the stress responsive gene is a gene responsive to salt stress.
[5] The expression enhancer according to any one of the above-described [1] to [3], wherein the stress responsive gene is a gene encoding a molecular chaperone.
[6] The expression enhancer according to any one of the above-described [1] to [3], wherein the stress responsive gene is a gene encoding one or more proteins selected from the group consisting of DnaJ and WRKY.
[7] A method for imparting stress resistance to a plant, comprising applying humic substances to a plant.
[8] The method according to the above-described [7], wherein humic substances are derived from lignite.
[9] The method according to the above-described [7] or [8], wherein the melanic index is 2.0 or higher.
[10] The method according to any one of the above-described [7] to [9], wherein the stress resistance is salt stress resistance.
[11] An expression enhancer for a gene involved in nitrogen metabolism of a plant, comprising humic substances.
[12] The expression enhancer according to the above-described [11], wherein humic substances are derived from lignite.
[13] The expression enhancer according to the above-described [11] or [12], wherein the melanic index is 2.0 or higher.
[14] The expression enhancer according to any one of the above-described [11] to [13], wherein the gene involved in nitrogen metabolism is a gene encoding a nitrate reductase.
[15] A photosynthesis activator comprising humic substances.
[16] The photosynthesis activator according to the above-described [15], wherein humic substances are derived from lignite.
[17] The photosynthesis activator according to the above-described [15] or [16], wherein the melanic index is 2.0 or higher.
[18] A method for activating the photosynthesis of a plant, comprising applying humic substances to a plant.
[19] The method according to the above-described [18], wherein humic substances are derived from lignite.
[20] The method according to the above-described [18] or [19], wherein the melanic index is 2.0 or higher.

### Advantageous Effects of Invention

Application of humic substances to a plant enhances the expression of a stress responsive gene in the plant, and imparts stress resistance to the plant. Application of humic substances enhances the expression of a gene involved in nitrogen metabolism in the plant and promotes growth of the plant. Application of humic substances to a plant enhances the photosynthetic rate in the plant and activate photosynthesis in the plant.

### Brief Description of Drawings

FIG. 1 is a diagram showing the gene expression in tomato leaves when humic substances are applied, with respect to the gene expression in tomato leaves when humic substances are not applied.
FIG. 2 is a diagram showing the expression of a gene encoding DnaJ (DnaJ/ActII) in tomato leaves when humic substances are not applied and when humic substances are applied.
FIG. 3 is a diagram showing the expression of a gene encoding NR (NR/ActII) in tomato leaves when humic substances are not applied and when humic substances are applied.

### Description of Embodiments

An expression enhancer for a stress responsive gene of a plant according to an embodiment of the present invention includes humic substances. Humic substances include one or more selected from the group consisting of humic substances and salts of humic substances. Examples of humic substances include natural humic substances produced in nature, such as peat and weathered coal; artificial humic substances artificially produced by oxidizing lignite with nitric acid, or the like; and salts of humic substances obtained by neutralizing natural humic substances or artificial humic substances with alkali substances such as sodium, potassium, ammonia, calcium, and magnesium. Examples of humic substances include humic acid, nitrohumic acid, ammonium humate, calcium humate, magnesium humate, ammonium nitrohumate, calcium nitrohumate, and magnesium nitrohumate.

Humic substances may be an extract of humic substances. An extract of humic substances refers to an extract obtained by extracting a nitric acid oxide of low rank coal such as lignite and brown coal at a pH in the range of 5 to 8, and preferably an extract extracted at a pH in the range of 5 to 7. The extract of humic substances is obtained as a liquid substance by, for example, stirring a mixture of a nitric acid oxide of low rank coal obtained by oxidizing and decomposing low rank coal with nitric acid (hereinafter, referred to as crude material of humic substances); inorganic compounds comprising at least one monovalent or divalent alkali selected from the group consisting of potassium hydroxide, sodium hydroxide, ammonium hydroxide, magnesium hydroxide, and calcium hydroxide; and water at 40°C to 90°C for 0.5 to 1 hour, and then carrying out a solid-liquid separation process. The inorganic compounds are added to water so as to make a pH in the range of 5 to 8. A production method for the extract of humic substances is described in Japanese Patent No. 6231059. From the viewpoint of the effect of enhancing the expression of a stress responsive gene, it is preferable that the extract of humic substances is a lignite-derived humic substances extract.

It is preferable that humic substances have a melanic index (MI) of 2.0 or higher. The MI is an index used for classifying humic substances and is the ratio of light absorbances at wavelengths of 450 nm and 520 nm (A450/A520) in the absorption spectrum of a sodium hydroxide extract (KUMADA Kyoichi, "Chemistry of Soil Organic Matter", 2nd edition, Gakkai Shuppan Center (1981); YAMAMOTO Sadahiro et al., "Easy Estimation of Humic Aid Types by Using Melanic Index", Japanese Journal of Soil Science and Plant Nutrition, Vol. 71, No. 1, p. 82-85 (2000)).

More specifically, the MI is calculated by the following method. A sample is pulverized using a mortar and a 250-µm sieve to obtain particles that pass through a 250-µm sieve. About 10 g of the particles are taken into a weighing bottle and precisely weighed. This weighing bottle is left to stand for about 12 hours in a dryer maintained at a temperature of 105°C, subsequently the temperature is returned to room temperature in a desiccator, and the weighing bottle is precisely weighed again. The mass decrement is regarded as moisture, and the water content percentage of the sample is determined. Next, 0.10 g, in an amount equivalent to dry mass, of the above-described particles that pass through a 250-µm sieve and 45 ml of a 0.5 mol/L aqueous solution of sodium hydroxide are added to a 50-ml centrifugal tube, the mixture is shaken at a rate of 250 rpm for about 1 hour at room temperature of 20°C, subsequently centrifugation is performed for about 10 minutes at 3,000 x g, and the supernatant thereof is filtered through a No. 5C filter paper manufactured by Advantec MFS, Inc. The absorbance at 450 nm and the absorbance at 520 nm of the filtrate are measured, using distilled water as a blank. In this case, when the absorbance at 450 nm shows a value of 1.0 or larger, a 0.1 mol/L aqueous solution of sodium hydroxide is added to the filtrate to adjust the absorbance to be 0.8 or higher and lower than 1.0, and then the absorbance at 520 nm is measured. The ratio of absorbance at 450 nm/ absorbance at 520 nm is calculated and is defined as MI.

The upper limit value of the MI is not particularly limited but can be set to 5.0 or less. Furthermore, the numerical value range of the MI is preferably 2.0 to 4.5, and more preferably 2.0 to 4.0.

The total organic carbon (TOC) concentration of the extract of humic substances is preferably 5,000 mg/L or more. The TOC concentration of the extract of humic substances is preferably 60,000 mg/L or less. The TOC concentration of the extract of humic substances is more preferably 10,000 to 50,000 mg/L.

A method for measuring the TOC concentration of an extract is defined as follows. The TOC concentration is a value obtained by making measurement using a supernatant obtained by centrifuging an extract of a crude material of humic substances at 3,000 x g, by a combustion catalytic oxidation method using a total organic carbon analyzer (TOC-L manufactured by SHIMADZU CORPORATION). When the extract contains non-humic substances such as urea, which are fertilizer components, substances (humic substances and fulvic acid fractions) separated in accordance with the method of the International Humic Substances Society (FUJITAKE, Humic Substances Research Vol. 3, p. 1-9) are quantitatively determined by the above-described technique, and the TOC concentration of the extract is measured.

The plant that is a target of the expression enhancer of a stress responsive gene of a plant according to the present embodiment may be a spermatophyte, a pteridophyte, or a bryophyte, the spermatophyte may be a gymnosperm or an angiosperm, and the angiosperm may be a monocotyledonous plant or may be a dicotyledonous plant. Furthermore, the spermatophyte includes plants that propagate without forming seeds, for example, vegetatively propagating plants that propagate by means of bulbs and tubers. From a commercial viewpoint, it is preferable that the targets of the expression enhancer include vegetables and flowering plants.

Specific examples of such plants include plants of the family Cruciferae, the family Solanaceae, the family Asteraceae, the family Cucurbitaceae, the family Umbelliferae, the family Gramineae, the family Rosaceae, the family Liliaceae, the family Orchidaceae, the family Amaryllidaceae, the family Primulaceae, the family Leguminosae, the family Allium, the family Polygonaceae, the family Convolvulaceae, the family Chenopodiaceae, the family Vitaceae, the family Rutaceae, the family Ebenaceae, the family Theaceae, the family Oleaceae, the family Malvaceae, the family Musaceae, the family Zingiberaceae, the family Rubiaceae, the family Bromeliaceae, and the like. Examples of the plants of the Cruciferae include Brassica campestris, bok choy, turnip, cauliflower, cabbage, Japanese radish, Chinese cabbage, and broccoli. Examples of the plants of the family Solanaceae include tomato, tobacco, sweet pepper, potato, red pepper, eggplant, paprika, and bell pepper. Examples of the plants of the family Asteraceae include lettuce, artichoke, burdock, garland chrysanthemum, chrysanthemum, and sunflower. Examples of the plants of the family Cucurbitaceae include pumpkin, cucumber, watermelon, and melon. Examples of the plants of the family Umbelliferae include water dropwort, celery, carrot, and parsley. Examples of the plants of the family Gramineae include rice, barley, wheat, sugar cane, and maize. Examples of the plants of the family Rosaceae include strawberry, rose, apple, pear, peach, loquat, and almond. Examples of the plants of the family Liliaceae include asparagus, tulip, and lily. Examples of the plants of the family Orchidaceae include orchid and cymbidium. Examples of the plants of the family Amaryllidaceae include daffodil. Examples of the plants of the family Primulaceae include cyclamen. Examples of the plants of the family Leguminosae include soybean, common beam, and adzuki bean. Examples of the plants of the family Allium include green onion, leek, scallion, and garlic. Examples of the plants of the family Polygonaceae include buckwheat. Examples of the plants of the family Convolvulaceae include sweet potato. Examples of the plants of the family Chenopodiaceae include spinach and sugar beet. Examples of the plants of the family Vitaceae include grapes. Examples of the plants of the family Rutaceae include mandarin orange, lemon, and orange. Examples of the plants of the family Ebenaceae include persimmon. Examples of the plants of the family Theaceae include tea. Examples of the plants of the family Oleaceae include olive and jasmine. Examples of the plants of the family Malvaceae include cotton, cacao, and okra. Examples of the plants of the family Musaceae include banana. Examples of the plants of the family Zingiberaceae include ginger. Examples of the plants of the family Rubiaceae include coffee tree. Examples of the plants of the family Bromeliaceae include pineapple and ananas.

The stress responsive gene is a gene whose expression is induced when stress is exerted on a plant. Examples of the stress responsive gene include genes encoding molecular chaperones such as DnaJ, HSP, and chaperonin; WRKY, universal stress protein, aquaporin, pathogenesis-related protein, and plant defensin. The present embodiment is useful for stress responsive genes encoding one or more proteins selected from the group consisting of DnaJ and WRKY.

A molecular chaperone (also referred to as chaperone) refers to a protein having, for example, a function of repairing a protein that does not correctly have a three-dimensional structure or the like, and the molecular chaperone plays an important role in the quality control of proteins. An example of the molecular chaperone useful for the present embodiment may be DnaJ.

WRKY refers to a family of plant transcription factors that regulate, for example, numerous processes in plants, including responses to biological and abiotic stress, immunity, plant defense, ageing, seed dormancy and germination, and the like.

In plants in a stressful environment, the expression (transcription and/or translation) of stress responsive genes is induced. When the expression enhancer for a stress responsive gene of a plant according to the present embodiment is applied to such a plant, the expression of the stress responsive gene is enhanced as compared to the case where the expression enhancer is not applied, and the amount of expression thereof is 1.1 or more folds, and preferably 1.5 or more folds, the amount of expression (amount of transcription and/or amount of translation) in the case where the expression enhancer for the stress responsive gene is not applied.

A method for imparting stress resistance to a plant according to an embodiment of the present invention comprises applying humic substances to a plant. Examples of the stress resistance include drought resistance, salt resistance, disease resistance, pest resistance, cold resistance, and high-temperature resistance; however, the stress resistance is preferably salt resistance. The site at which humic substances are used is not particularly limited and can be set to the rhizosphere. The application amount and the application period of humic substances are not particularly limited, and in the case of soil application, 0.1 to 5000 mg/L as the total organic carbon concentration can be applied 1 to 12 times a month; in the case of hydroponic cultivation, 0.1 to 5000 mg/L as the total organic carbon concentration can be applied 1 to 12 times a month; and in the case of leaf surface application, 0.1 to 5000 mg/L as the total organic carbon concentration can be applied 1 to 12 times a month.

The expression enhancer for a gene involved in nitrogen metabolism of a plant according to an embodiment of the present invention comprises humic substances. Humic substances, the plant as a target, and the like are as described above.

The gene involved in nitrogen metabolism is a gene encoding a protein involved in a reaction of converting nitrogen or a nitrogen compound to another nitrogen compound, and examples include genes encoding a nitrate reductase (NR) and a nitrate ion transporter (Nrt).

When the expression enhancer for a gene involved in nitrogen metabolism of a plant according to an embodiment of the present invention is applied to a plant, the expression of the gene involved in nitrogen metabolism is enhanced as compared to the case where the expression enhancer is not applied, and the amount of expression thereof is 1.1 or more folds, and preferably 1.5 or more folds, the amount of expression (amount of transcription and/or amount of translation) in the case where the expression enhancer for a gene involved in nitrogen metabolism is not applied.

A photosynthesis activator according to an embodiment of the present invention comprises humic substances. Humic substances, the plant as a target, and the like are as described above.

When the photosynthesis activator according to the present embodiment is applied to a plant, photosynthesis is activated as compared to the case where the photosynthesis activator is not applied, and the photosynthetic rate is 1.1 or more folds, and preferably 1.3 or more folds, the photosynthetic rate in the case where the photosynthesis activator is not used.

A method for activating photosynthesis of a plant according to an embodiment of the present invention comprises applying humic substances to a plant. The site of applying humic substances, the application amount, and the application period are as described above. When humic substances are applied to a plant, photosynthesis of the plant is activated, the expression of a gene involved in nitrogen metabolism is enhanced, and at the same time, growth of the plant is promoted.

### EXAMPLES

### Test Example 1: Cultivation of tomato under salt stress

1. Tomato (Micro-Tom) seeds were taken out of a refrigerator and left to stand overnight at room temperature (about 25°C).
2. Wetted paper towel was placed on a plastic Petri dish, and tomato seeds were seeded thereon at an interval of about 1 cm. Thereafter, the Petri dish was left to stand in an incubator.
3. Four days after seeding, germinated tomato seeds were transplanted into rockwool (Grodan B.V.) and arranged on trays, and then OAT A Formula (OAT Agrio Co., Ltd.; total nitrogen 260 ppm, P₂O₅ 120 ppm, K₂O 405 ppm) was applied.
4. One week after the transplantation, OAT A Formula was applied at a TOC concentration of 8 ppm to zones where humic substances were to be applied, and humic substances were acclimatized. In zones where humic substances were not to be applied, only the OAT A Formula was applied.
5. One week after the humic substance acclimatization, the plants were transferred to a plastic container from each test zone, and a salt stress test was carried out. The test conditions were as described in the section "Test zone" that will be described below.
6. The medium was exchanged at an interval of one week from the initiation of the salt stress test.
7. For the growth investigation, the maximum leaf length (cm), the number of set flowers (pieces), SPAD, and the main stem length (cm) were measured. The SPAD (chlorophyll content included in the leaves) was measured using SPAD-502 Plus manufactured by Konica Minolta, Inc.

Cultivation was carried out in an incubator (TOKYO RIKAKIKAI CO., LTD.; FLI-2010H-LED) in all cases. The incubation conditions are as follows.
Day length: 16 hours
Temperature: 25°C, 16 hours/20°C, 8 hours

### Test zone

With regard to a salt stress test, the NaCl concentration was adjusted to 0, 50 mM, or 100 mM, and the test was carried out without applying humic substances or by applying humic substances (TOC concentration 15 or 60 ppm). With regard to a gene expression analysis and a photosynthetic rate measurement test, the tests were carried out without applying humic substances or by applying humic substances (TOC concentration 60 ppm).

### Humic substances

The humic substances used was a lignite-derived humic substances extract, and the TOC and MI were adjusted by adjusting the amount of nitric acid to be added during the production of the humic substances extract, in accordance with the method described in Japanese Patent No. 6231059. The humic substances used were Product 1 (lignite-derived humic substances extract, TOC 35000 ppm, MI 2.0 or higher) and Product 2 (lignite-derived humic substances extract, TOC 20000 ppm, MI 4.0).

The maximum leaf length, the number of set flowers, the SPAD, and the main stem length could be increased by applying humic substances, and growth promotion and salt stress resistance enhancement in tomatoes were verified. As an example, the results obtained by cultivating tomatoes for five weeks under the conditions of salt stress at a NaCl concentration of 100 mM by applying Product 1 at a concentration of 15 ppm are shown in the following table.

**[Table 1]**

| | Test zone (N = 5) | | | |
|---|---|---|---|---|
| | Blank | Humic substances | NaCl | Humic substances + NaCl |
| Maximum leaf length (cm) | 6.9 ± 0.4 | 7.3 ± 0.6 | 5.1 ± 0.4 | 5.5 ± 0.6 |
| Number of set flowers (pieces) | 34.6 ± 11.2 | 45.6 ± 16.5 | 11.6 ± 3.1 | 16.2 ± 3.3 |
| SPAD | 51.8 ± 1.4 | 52.7 ± 1.6 | 39.3 ± 1.3 | 40.2 ± 2.9 |
| Main stem length (cm) | 9.0 ± 0.6 | 9.1 ± 1.1 | 7.1 ± 1.6 | 7.5 ± 1.2 |

### Test Example 2: Microarray analysis

### Gene expression analysis

Extraction of a transcriptional product was carried out as follows.
1. Humic substances were applied, and after 24 hours, leaves were picked with tweezers and rapidly frozen with liquid nitrogen.
2. The frozen tomato leaves were subjected to bead pulverization, and a transcriptional product was extracted. Pulverization was performed using Precellys Evolution (Bertin Technologies SAS) and MN Bead Tubes Type G (Takara Bio Inc.).
3. Purification of the transcriptional product was carried out using Cica geneus (registered trademark) RNA Prep Kit (for plants) (KANTO CHEMICAL CO., INC.). At that time, contamination of DNA in the transcriptional product was checked by measurement of Abs260/Abs280 or the like.
4. The obtained transcriptional product was subjected to a reverse transcription reaction (ReverTra Ace (registered trademark) qPCR RT Master Mix with gDNA Remover; TOYOBO CO., LTD.) to synthesize cDNA, and a gene expression analysis was performed.

A microarray analysis was carried out as follows.
1. An analysis using Tomato Gene Expression Microarray (Agilent Technologies, Inc.) was carried out in Takara Bio Inc.
2. The data were analyzed using a Platform of Subio inc. A gene that exhibited expression variation twice or more as compared to the case where humic substances were not applied, were considered as genes whose expression was affected by humic substances.

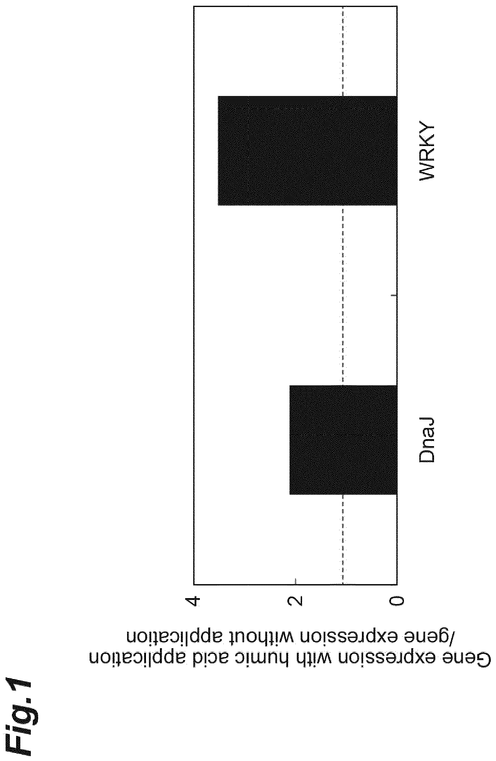

FIG. 1 is a diagram showing the gene expression in tomato leaves when humic substances are applied, with respect to the gene expression in tomato leaves when humic substances are not applied. An enhancement of the expression of genes encoding DnaJ and WRKY, which are stress responsive genes, was verified.

### Test Example 3: Quantitative PCR analysis

A quantitative PCR analysis was carried out as follows.
1. When the ACT2 gene was used as a housekeeping gene, the expression of the gene encoding DnaJ, a gene encoding a molecular chaperone was increased in the microarray, and growth became vigorous, suggesting that nitrogen metabolism became active. Therefore, the expression of the gene encoding NR involved in nitrate reduction was investigated. The ACT2 gene refers to one of actin genes. The instrument used was QuantStudio 3 (Applied Biosystems, Inc.), and the reagent used was KOD SYBR (registered trademark) qPCR Mix (TOYOBO CO., LTD.). The sequences of the used primers are shown below.

### ACT2

cattgtgctcagtggtggttc (SEQ ID NO: 1)
tctgctggaaggtgctaagtg (SEQ ID NO:2) DnaJ
ctacgatccaggagatcaag (SEQ ID NO:3)
gacgtgtccttctgatcaat (SEQ ID NO:4) NR
cgtaggccgtactttcaagc (SEQ ID NO:5)
catcgtcatcctcgtcttca (SEQ ID NO:6)

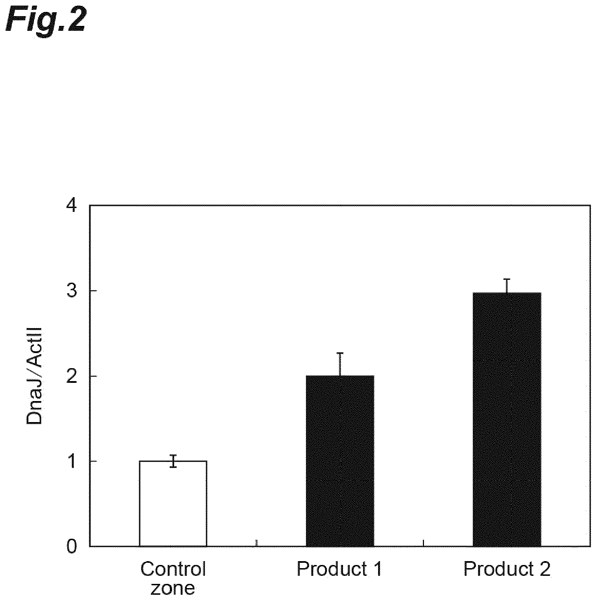

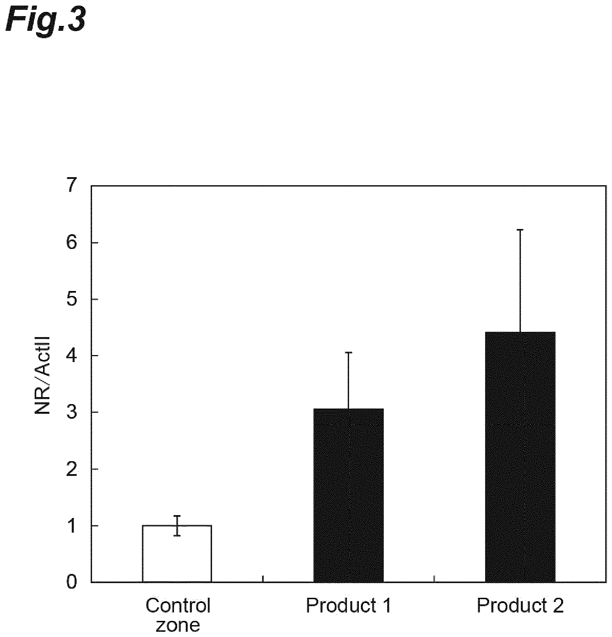

FIG. 2 is a diagram showing the expression of a gene encoding DnaJ (DnaJ/ActII) in tomato leaves when humic substances are not applied and when humic substances are applied. When humic substances were used, the expression of the gene encoding DnaJ was enhanced to 2.0 folds. FIG. 3 is a diagram showing the expression of a gene encoding NR (NR/ActII) in tomato leaves when humic substances are not applied and when humic substances are applied. When humic substances were used, the expression of the gene encoding NR was enhanced to 2.5 folds.

### Test Example 4: Photosynthetic rate analysis

Based on the results showing that growth became more vigorous when humic substances were applied were obtained, it was speculated that photosynthesis became active, and the photosynthetic rate was measured in the third week and the fourth week of cultivation. It was verified that the photosynthetic rate increases when humic substances are applied. As an example, the results obtained when Product 2 was applied are shown in the following table.

Photosynthetic transpiration measurement and chlorophyll fluorescence measurement were carried out.

For the photosynthetic transpiration measurement, the transpiration rate, the photosynthetic rate, and the stomatal conductance were measured using a plant photosynthesis comprehensive analysis system (manufactured by Li-COR, Inc., LI-6800). The transpiration rate is a rate at which the moisture produced by a photosynthetic reaction is transpired from the leaves. Regarding the transpiration rate, the value increases as the photosynthetic reaction proceeds more satisfactorily. The stomatal conductance is a value that serves as an index of the degree of opening of stomata for taking in carbon dioxide into the leaves. As the stomatal conductance is larger, it is easier to take in carbon dioxide into the leaves, and the photosynthetic reaction is likely to proceed satisfactorily.

For the chlorophyll fluorescence measurement, the effective quantum yield, the electron transfer rate, and the non-photochemical quenching were measured using a plant photosynthesis comprehensive analysis system (manufactured by Li-COR, Inc., LI-6800). Non-photochemical quenching (NPQ) is one of photoprotection mechanisms and is an index representing the mechanism of dissipating excessively absorbed irradiation as heat. A smaller value of the non-photochemical quenching implies that even if thermal dissipation is not exhibited highly, light can be sufficiently utilized so that the photosynthesis efficiency of the plant and the plant growth are high.

It was verified that when humic substances are used, the transpiration rate, the photosynthetic rate, the stomatal conductance, the effective quantum yield, and the electron transfer rate are increased, the value of the non-photochemical quenching is decreased, and the photosynthesis efficiency and plant growth are high.

**[Table 2]**

| | Photosynthetic transpiration measurement | | |
|---|---|---|---|
| Measurement item | Transpiration rate | Photosynthetic rate | Stomatal conductance |
| Unit | mol m⁻² s⁻¹ | µmol m⁻² s⁻¹ | mol m⁻² s⁻¹ |
| Blank | 0.00314 | 11.723 | 0.227 |
| Product 2 | 0.00405 | 15.688 | 0.299 |

| | Chlorophyll fluorescence measurement | | |
|---|---|---|---|
| Measurement item | Effective quantum yield | Electron transfer rate | Non-photoche mical quenching |
| Unit | | µmol m⁻² s⁻¹ | |
| Blank | 0.237 | 99.918 | 1.536 |
| Product 2 | 0.268 | 112.83 | 0.858 |

## Claims

1. An expression enhancer for a stress responsive gene of a plant, comprising humic substances.

2. The expression enhancer according to claim 1, wherein humic substances are derived from lignite.

3. The expression enhancer according to claim 1 or 2, wherein the melanic index is 2.0 or higher.

4. The expression enhancer according to any one of claims 1 to 3, wherein the stress responsive gene is a gene responsive to salt stress.

5. The expression enhancer according to any one of claims 1 to 3, wherein the stress responsive gene is a gene encoding a molecular chaperone.

6. The expression enhancer according to any one of claims 1 to 3, wherein the stress responsive gene is a gene encoding one or more proteins selected from the group consisting of DnaJ and WRKY.

7. A method for imparting stress resistance to a plant, comprising applying humic substances to the plant.

8. The method according to claim 7, wherein humic substances are derived from lignite.

9. The method according to claim 7 or 8, wherein the melanic index is 2.0 or higher.

10. The method according to any one of claims 7 to 9, wherein the stress resistance is salt stress resistance.

11. An expression enhancer for a gene involved in nitrogen metabolism of a plant, comprising humic substances.

12. The expression enhancer according to claim 11, wherein humic substances are derived from lignite.

13. The expression enhancer according to claim 11 or 12, wherein the melanic index is 2.0 or higher.

14. The expression enhancer according to any one of claims 11 to 13, wherein the gene involved in nitrogen metabolism is a gene encoding a nitrate reductase.

15. A photosynthesis activator comprising humic substances.

16. The photosynthesis activator according to claim 15, wherein humic substances are derived from lignite.

17. The photosynthesis activator according to claim 15 or 16, wherein the melanic index is 2.0 or higher.

18. A method for activating photosynthesis of a plant, comprising applying humic substances to a plant.

19. The method according to claim 18, wherein humic substances are derived from lignite.

20. The method according to claim 18 or 19, wherein the melanic index is 2.0 or higher.
